# EUROPÄISCHE PATENTSCHRIFT

(11) **EP 0 835 098 B1**
(45) Veröffentlichungstag und Bekanntmachung des Hinweises auf die Patenterteilung: **04.09.2002**
(21) Anmeldenummer: 96923883.1
(22) Anmeldetag: 21.06.1996
(51) Int. Cl.: A61K 9/12, A61K 31/58, A61K 31/46

(54) **NEUE STABILE ARZNEIMITTELZUBEREITUNG ZUR ERZEUGUNG TREIBGASFREIER AEROSOLE**
NEW, STABLE MEDICINAL COMPOSITIONS FOR GENERATING PROPELLANT-FREE AEROSOLS
NOUVELLES COMPOSITIONS MEDICAMENTEUSES STABLES UTILES POUR GENERER DES AEROSOLS SANS GAZ PROPULSEURS

(30) Priorität: 27.06.1995 DE 19523207
(43) Veröffentlichungstag der Anmeldung: 15.04.1998
(73) Patentinhaber: Boehringer Ingelheim Pharma KG, 55218 Ingelheim am Rhein (DE)
(72) Erfinder: FREUND, Bernhard, D-55435 Gau-Algesheim (DE); KRÜGER, Michael, D-55218 Ingelheim am Rhein (DE); ZIERENBERG, Bernd, D-55411 Bingen am Rhein (DE)
(74) Vertreter: Laudien, Dieter, Dr.
(86) Internationale Anmeldenummer: EP9602700
(87) Internationale Veröffentlichungsnummer: WO97001329

(56) Entgegenhaltungen:
- EP-A- 0 234 500
- EP-A- 0 310 910
- EP-A- 0 372 777
- WO-A-92/06675
- WO-A-94/13263
- DE-A- 2 926 095
- DE-A- 4 123 663
- US-A- 5 047 230
- US-A- 5 370 862
- Patent Abstracts of Japan, Band 10, Nr 252(C-369); & JP,A,6183117 (SUMITOMO CHEM CO LTD), 1986-04-26

## Beschreibung

Die vorliegende Erfindung betrifft Arzneimittelzubereitungen in Form von stabilen ethanolischen Wirkstofflösungen zur Erzeugung treibgasfreier Aerosole.

Die Anwendung von Dosieraerosolen ist in den letzten 20 Jahren fester Bestandteil bei der Therapie obstruktiver Lungenerkrankungen, insbesondere von Asthma, gewesen. Üblicherweise wurden als Treibgase Fluorchlorkohlenwasserstoffe eingesetzt. Nachdem das ozonschädigende Potential dieser Treibgase erkannt worden war, wurden vermehrt Anstrengungen unternommen, Alternativen hierzu zu entwickeln. Als eine Alternative bietet sich die Entwicklung von Verneblern an, bei denen wässerige Lösungen pharmakologisch aktiver Stoffe unter hohem Druck so versprüht werden, daß Nebel inhalierbarer Teilchen entstehen. Der Vorteil dieser Vernebler ist, daß auf den Einsatz von Treibgasen völlig verzichtet werden kann.

Solche Vernebler sind beispielsweise in der PCT-Patentanmeldung WO91/14468 beschrieben, auf die hiermit inhaltlich Bezug genommen wird. Bei den dort beschriebenen Verneblern werden wirkstoffhaltige Lösungen definierter Volumina unter Anwendung hoher Drucke durch kleine Düsen versprüht, so daß inhalierbare Aerosole mit einer bevorzugten Teilchengröße zwischen 1 und 10, bevorzugt zwischen 2 und 5 Mikrometer entstehen.

Bislang wurde davon ausgegangen, daß bei herkömmlichen treibgashaltigen Dosieraerosolen ein Optimum an lungengängigen Teilchen im Aerosol erhalten wird. Überraschenderweise wurde jetzt gefunden, daß durch die Verwendung von ethanolischen Wirkstofflösungen in Kombination mit den oben genannten Verneblern wesentlich bessere inhalierfähigere Teilchenspektren generiert werden können, als dies üblicherweise bei treibgashaltigen Dosieraerosolen der Fall ist.

Als Lösungsmittel für die Arzneimittelzubereitung im Sinne der vorliegenden Erfindungen sind Lösungen geeignet, die mindestens 70 % (v/v) Ethanol enthalten, bevorzugt sind Lösungen mit mindestens 85 % (v/v), besonders bevorzugt sind Lösungen mit einem Ethanolgehalt von über 95 % (v/v). Die Konzentrationsangabe erfolgt in Volumen Prozent (v/v), wobei der Rest Wasser ist. Ganz besonders bevorzugt ist Ethanol, das bereits geringe Mengen Wasser enthält - beispielsweise 96%iges Ethanol - so daß es nicht mehr hygroskopisch ist und azeotrop verdampft.

Weitere Bestandteile des Lösungsmittels sind neben Wasser gegebenenfalls weitere Cosolventien, ebenfalls kann die Arzneimittelzubereitung Geschmackstoffe und weitere pharmakologische Hilfsstoffe enthalten. Beispiele für Cosolventien sind solche, die Hydroxylgruppen oder andere polare Gruppen enthalten, beispielsweise Alkohole - insbesondere Isopropylalkohol, Glykole - insbesondere Propylenglykol, Polyethylenglykol, Polypropylenglykol, Glykolether, Glycerol, Polyoxyethylenalkohole und Polyoxyethylen-Fettsäureester. Cosolventien sind dazu geeignet, die Löslichkeit von Hilfsstoffen und gegebenenfalls der Wirkstoffe zu erhöhen.

Der Anteil des gelösten Arzneistoffes an der fertigen Arzneimittelzubereitung beträgt zwischen 0.001 und 5 % - vorzugsweise zwischen 0.05 bis 3%, insbesondere 0.01- bis 2 % wobei sich die Angaben auf Gew. % beziehen. Die maximale Konzentration des Arzneistoffes ist abhängig von der Löslichkeit im Lösungsmittel und von der erforderlichen Dosierung zur Erzielung der gewünschten therapeutischen Wirkung.

Als Arzneistoffe in den neuen Zubereitungen werden Substanzen verwendet die für die inhalative Anwendung geeignet sind und in dem vorgegebenen Lösungsmittel löslich sind, ausewählt aus der Grüppe bestehend aus Betamimetica, Anticholinergika, Antiallergika, PAF-Antagonisten und insbesondere um Steroide sowie Wirkstoffkombinationen davon.

Im einzelnen seien als Beispiele genannt:

Tiotropiumbromid, 3-[(hydroxydi-2-thienylacetyl)oxy]-8,8-dimethyl-,8-azoniabicyclo[3.2.1]oct-6-en-bromid

### Als Betamimetika:

| | | | |
|---|---|---|---|
| Bambuterol | Bitolterol | Carbuterol | Formoterol |
| Clenbuterol | Fenoterol | Hexoprenalin | Procaterol |
| Ibuterol | Pirbuterol | Salmeterol | Tulobuterol |
| Reproterol | Salbutamol | Sulfonterol | Terbutalin |

1-(2-Fluor-4-hydroxyphenyl)-2-[4-(1-benzimidazolyl)-2-methyl-2-butylamino]ethanol, erythro-5'-Hydroxy-8'-(1-hydroxy-2-isopropylaminobutyl)-2H-1,4-benzoxazin-3-(4H)-on,
1-(4-Amino-3-chlor-5-trifluormethylphenyl)-2-tert.-butyl-amino)ethanol, 1-(4-Ethoxycarbonylamino-3-cyan-5-fluorphenyl)-2-(tert.-butylamino)ethanol.

### Als Anticholinergika:

Ipratropiumbromid
Oxitropiumbromid
Trospiumchlorid
Benzilsäure-N-β-fluorethylnortropinestermethobromid

### Als Steroide:

Budesonid
Beclometason (bzw. das 17,21-Dipropionat)
Dexamethason-21-isonicotinat
Flunisolid

### Als Antiallergika:

Dinatriumcromoglicat
Nedocromil
Epinastin

### Als PAF-Antagonisten:

WEB 2086 (4-(2-Chlorphenyl)-9-methyl-2-[3(4-morpholinyl)-3-propanon-1-yl]-6Hthieno-[3,2-f][1,2,4]triazolo[4,3-a][1,4]diazepin )
WEB 2170
   (6-(2-Chlorphenyl)-8,9-dihydro-1-methyl-8-[(4-morpholinyl)carbonyl]-4H,7H-cyclopenta-[4,5]thieno-[3,2-f][1,2,4]triazolo[4,3-a][1,4]diazepin )

Die erfindungsgemäßen Arzneimittelzubereitungen können weitere Hilfsstoffe enthalten, beispielsweise Sojalecithin, oberflächenaktive Stoffe.

Überraschenderweise hat sich gezeigt, daß der Zusatz einer organischen oder anorganischen Säure, bevorzugt in Kombination mit einem Komplexbildner, zu einer Verbesserung der Stabilität (Lagerstabilität) von steroidhaltigen, insbesondere von Arzneimittelzubereitungen, die Flunisolid bzw. seines Hydrates bzw. Hemihydrates oder Budenosid als Wirkstoff enthalten, führt, die Ethanol als Lösungsmittel enthalten.

Als anorganische Säuren werden beispielhaft genannt: Salzsäure, Schwefelsäure oder Phosphorsäure; als Beispiele für organische Säuren werden genannt: Ascorbinsäure, Zitronensäure, Apfelsäure, Weinsäure, Maleinsäure, Bersteinsäure, Fumarsäure, Essigsäure, Ameisensäure, Propionsäure u. andere.

Die Menge an Säure in der fertigen Arzneimittelzubereitung wird in jedem Fall so gewählt, daß der pH-Wert der Lösung zwischen 2.0 und 7.0, insbesondere zwischen 3.0 und 4.0 beträgt.

In einer bevorzugten Ausführungsform enthält die Arzneimittelzubereitung weiterhin einen Komplexbildner. Als Komplexbildner werden beispielhaft genannt EDTA, Zitronensäure, Nitrilotriessigsäure und deren Salze. Die Menge an Komplexbildner beträgt zwischen 0.1 und 3 mg/100 ml, bevorzugt zwischen 0.2 und 2 mg/100 ml, insbesondere zwischen 0.9 und 1.1 mg/100 ml bezogen auf die fertige Arzneimittelzubereitung.

Bevorzugter Komplexbildner ist EDTA (Ethylendiamintetraessigsäure, bzw ein Salz davon, wie beispielsweise das Dinatriumsalz). Eine bevorzugte Arzneimittelzubereitung der vorliegenden Erfindung enthält 1,667 % Flunisolide im Ethanol (96 % v/v) als Lösungsmittel die 0.01 % (v/v) EDTA als Komplexbildner enthält und durch Zusatz von Säure auf einen pH zwischen 3.0 und 4.0 eingestellt ist.

Beispiele für Steroide, die als Wirkstoff in der erfindungsgemäßen Arzneimittelzubereitung verwendet, werden können sind:

| | |
|---|---|
| Seratrodast | Mycophenolate mofetil |
| Pranlukast | Zileuton |
| Butixocort | Budesonide |
| Deflazacort Fluticasone | Promedrol |
| Mometasone furoate | Tipredane |
| Beclomethasone, Douglas | Icomethasone enbutate |
| Ciclometasone | Cloprednol |
| Fluocortin butyl | Halometasone |
| Deflazacort | Alclometasone |
| Ciclometasone | Alisactide |
| Prednicarbate | Hydrocortison-butyratpropionat |
| Tixocortol-pivalate | Alclometasone-dipropionate |
| Lotrisone | Canesten-HC |
| Deprodone | Fluticasone-propionate |
| Methylprednisolone-Aceponate | Halopredone-acetate |
| Mometasone | Mometasone-furoate |
| Hydrocortisone-aceponate | Mometasone |
| Ulobetasol-propionate | Aminoglutethimide |
| Triamcinolone | Hydrocortisone |
| Meprednisone | Fluorometholone |
| Dexamethasone | Betamethasone |
| Medrysone | Fluclorolone acetonide |
| Fluocinolone acetonide | Paramethasone-acetate |
| Deprodone Propionate | Aristocort-diacetat |
| Fluocinonide | Mazipredone |
| Difluprednate | Betamethasoe valerate |
| Dexamethasonisonicotinat | Beclomethasone-Dipropionate |
| Fluocortoloncapronat | Formocortal |
| Triamcinolon-Hexacetonide | Cloprednol |
| Formebolone | Clobetason |
| Endrisone | Flunisolide |
| Halcinonide | Fluazacort |
| Clobetasol | Hydrocortison-17-Butyrat |
| Diflorasone | Fluocortin |
| Amcinonide | Betamethasone Dipropionate |
| Cortivazol | Betamethasonadamantoat |
| Fluodexan | Trilostane |
| Budesonide | Clobetasone |
| Demetex | Trimacinolon Benetonid |

9.alpha.-chloro-6.alpha.-fluoro-11.beta.17.alpha.-dihydroxy-16.alpha.-methyl-3-oxo-1,4-androstadiene-17.beta.-carboxylic acid-methylester-17-propionate.

Die Tabelle 1 zeigt einen Vergleich einer Depositionsstudie, die zum einen mit einem handelsüblichen Dosieraerosol Inhacort® (Flunisolide, Dichlormethan, Trichlorfluormethan, Cryofluoran, Sorbitantriolat) = MDI und zum anderen mit der erfindungsgemäßen Arzneimittelzubereitung enthaltend Flunisolid in 96 % (v/v) Ethanol, die mit einem Vernebler wie aus der oben genannten PCT-Anmeldung WO 91/14468 (BINEB®^{:} technische Daten: Volumen der applizierten Arzneimittelzubereitung 15 µl, Druck ca. 300 bar, 2 Strahlen impaktiert aus zwei Düsenöffnungen der Größe 5 x 8 µm) durchgeführt wurde.

**Tabelle I**

| Tabelle I: Depositionsstudie | | |
|---|---|---|
| | BINEB® | MDI |
| Lunge (%) | 39.7 (9.9) | 15.3 (5.1) |
| Mundstück (%) | 39.9 (9.4) | 66.9 (7.1) |
| Exhalierter Anteil (%) | 10.4 (4.9) | 1.4 (1.3) |
| | | |
| Zentraler Lungenbereich (%) | 10.7 (2.5) | 4.5 (1.8) |
| mittlerer Lungenbereich (%) | 14.9 (3.6) | 5.4 (1.9) |
| peripherer Lungenbereich (%) | 14.1 (4.3) | 5.4 (1.4) |
| | | |
| Peripheral zone / Central zone ratio | 1.3 (0.2) | 1.3 (0.2) |

Die Tabelle zeigt eindeutig den Vorteil der erfindungsgemäßen Arzneimittelzubereitung, die mit dem beschriebenen Vernebler appliziert wurden.

### Beispiele:

Flunisolid hemihydrat-6α-Fluoro-11β,16α,17α,21-tetrahydropregna-1,4-diene-3,20-16 acetonide hemihydrate weist ein Molekulargewicht von 442,5. Bei der Anwendung im BINEB werden pro Dosierung 250 µg Flunisolid in 15 ul Lösungsmittel gelöst, so daß sich eine Konzentration von 1,667% (g/100 ml) ergibt.

Als Lösungsmittel wird 96%iges Ethanol verwendet. Zusätzlich enthält die fertige Arzneimittelzubereitung 1mg/100 ml Dinatrium-EDTA. Der pH-Wert der Arzneimittelzubereitung wird mit 0.1 N HCI auf pH 4 eingestellt.

In Analogie zu obigem Versuch wurden Formulierungen hergestellt, die Budesonid als Wirkstoff enthalten.

Es wurden folgende Mischungen von Arzneimittelzubereitungen hergestellt, die Flunisolid-hemihydrat als Wirkstoff enthalten.

**Tabelle II**

| Versuch Nr. | Kombination | Gehalt an Ethanol in (v/v) % | pH-Wert | Menge an Dinatrium EDTA in mg/100 ml |
|---|---|---|---|---|
| 1 | 1 | 85 | 3.6 | 0 |
| 2 | A | 96 | 3.6 | 0 |
| 3 | B | 85 | 7.0 | 0 |
| 4 | A B | 96 | 7.0 | 0 |
| 5 | C | 85 | 3.6 | 1 |
| 6 | A C | 96 | 3.6 | 1 |
| 7 | B C | 85 | 7.0 | 1 |
| 8 | A B C | 96 | 7.0 | 1 |

Der Gehalt an Flunisolid-hemihydrat betrug 1.666,7 mg/100 ml. Der pH-Wert der Lösung wurde mit 1 N HCI eingestellt und mit einem pH-Meter, pH 1162-Radiometer Kopenhagen, bestimmt. Die Proben werden in 5 ml Glasampullen abgefüllt und bei 80°C unter Lichtausschluß gelagert. Die Kombination AC zeigte nach 30 Tagen Lagerung die geringste Menge an Zersetzungsprodukt.

Weitere Formulierungsbeispiele die zusätzlich Dinatrium EDTA als Komplexbildner enthalten sind in Tabelle III dargestellt.

**Tabelle III**

| Bestandteile | I Menge in mg/100 ml | II Menge in mg/100 ml | III Menge in mg/100 ml | IV Menge in mg/100 ml |
|---|---|---|---|---|
| Flunisolid hemihydrat | 1667 | 1667 | 1667 | 1667 |
| Disodium EDTA | 1 | 1 | 1 | 1 |
| 0.1 n HCl | ad pH 3.6 | ad pH 3.2 | ad pH 4.0 | ad pH 3.6 |
| Menthol | - | - | - | 667 |
| Ethanol 96 % | ad 100 ml | ad 100 ml | ad 100 ml | ad 100 ml |

Der Hilfsstoff Menthol wurde zugesetzt, um bei Bedarf den bitteren Geschmack des Steroids zu überdecken.

Die oben beschriebenen Formulierungen wurden in 5 ml Glasampullen abgefüllt und bei 80°C gelagert. Bevorzugt - aufgrund der geringen Menge Zersetzungsprodukt - ist die Zubereitung III.

In der Tabelle IV sind Formulierungsbeispiele für Budenosid dargestellt.

**Tabelle IV**

| Bestandteile | I Menge in mg/100 ml | II Menge in mg/100 ml | III Menge in mg/100 ml | IV Menge in mg/100 ml | V Menge in mg/100 ml |
|---|---|---|---|---|---|
| Budesonid | 1333 | 1333 | 1333 | 1333 | 1333 |
| Disodium EDTA | 1 | - | 1 | 1 | - |
| 0.1 n HCL ad pH | 3.2 | 3.2 | 3.6 | 4.0 | 4.0 |
| Ethanol 96 % | ad 100 | 100 | 100 | 100 | 100 |

Nach einer Lagerung von 3 Monaten bei 80°C in verschlossenen Glasampullen wurde die Menge an Zersetzungsprodukten per HPLC-Methode bestimmt. Die Formulierungen IV und V zeigten die geringste Menge an Zersetzungsprodukt.

## Patentansprüche

1. Arzneimittelzubereitung zur Erzeugung treibgasfreier, inhalierbarer, lungengängiger Aerosole in Form einer Wirkstofflösung, dadurch charakterisiert, dass ein aktiver Wirkstoff aus der Gruppe der Betamimetica, Anticholinergika, Antiallergika, PAF-Antagonisten, der Steroide sowie Wirkstoffkombinationen davon zu 0,001 bis 5,0 Gewichtsprozent gegebenenfalls zusammen mit Geschmackstoffen und/oder pharmakolgisch unbedenklichen Hilfsstoffen in einem treibgasfreien Lösungsmittel gelöst wird, das zu wenigstens 70 Volumenprozent aus Ethanol und ansonsten aus Wasser sowie gegebenenfalls Cosolventien besteht.

2. Arzneimittelzubereitung nach Anspruch 1, dadurch charakterisiert, dass der aktiver Wirkstoff aus der Gruppe der Betamimetica, Anticholinergika und/oder Antiallergika ausgewählt ist.

3. Arzneimittelzubereitung nach Anspruch 1 oder 2, dadurch charakterisiert, dass das Lösungsmittel wenigstens 85 Volumenprozent Ethanol enthält.

4. Arzneimittelzubereitung nach einem der Ansprüche 1, 2 oder 3, dadurch charakterisiert, dass das Lösungsmittel wenigstens 95 Volumenprozent Ethanol enthält.

5. Arzneimittelzubereitung nach einem der Ansprüche 1- 4, dadurch charakterisiert, dass die Lösung neben dem Wirkstoff ein oder mehrere pharmakologisch verträgliche Hilfsstoffe oder Geschmacksstoffe enthält.

6. Arzneimittelzubereitung nach Anspruch 5, dadurch charakterisiert, dass das Lösungsmittel wenigstens 85 Volumenprozent Ethanol enthält und die Arzneimittelzubereitung einen Komplexbildner enthält.

7. Arzneimittelzubereitung nach Anspruch 6, dadurch charakterisiert, dass das Lösungsmittel wenigstens 96 Volumenprozent Ethanol enthält und der Komplexbildner EDTA oder ein Salz davon ist.

8. Arzneimittelzubereitung nach einem der Ansprüche 1- 7, dadurch charakterisiert, dass die aktive Substanz Tiotropiumbromid oder ein Säureadditionssalz davon ist.

9. Arzneimittelzubereitung nach einem der Ansprüche 1- 7, dadurch charakterisiert, dass die aktive Substanz 3-[Hydroxydi-2-thienylacetyl)oxy]-8,8-dimethyl-8-azoniabicyclo[3,2,1]oct-6-en oder ein Säureadditionssalz davon ist.

10. Arzneimittelzubereitung nach einem der Ansprüche 1- 5 dadurch charakterisiert das die aktive Substanz Ipratropiumbromid, Oxitropiumbromid oder Trospiumchlorid ist.

11. Arzneimittelzubereitung nach einem der Ansprüche 1, 3, 4 oder 5, dadurch charakterisiert, dass der Wirkstoff ein Steroid ist und die Arzneimittelzubereitung eine organische oder anorganische Säure enthält.

12. Arzneimittelzubereitung nach Anspruch 11, dadurch charakterisiert, dass das Lösungsmittel zu wenigstens 96 Volumenprozent Ethanol enthält.

13. Arzneimittelzubereitung nach Anspruch 11 oder 12, dadurch charakterisiert, dass die Arzneimittelzubereitung einen Komplexbildner enthält.

14. Arzneimittelzubereitung nach Anspruch 13, dadurch charakterisiert, dass der Komplexbildner EDTA oder ein Salz davon ist.

15. Arzneimittelzubereitung nach einem der Ansprüche 11, 12, 13 oder 14, dadurch charakterisiert, dass die Menge des Komplexbildners zwischen 0,1 und 5 mg pro 100 ml Lösungsmittel beträgt.

16. Arzneimittelzubereitung nach einem der Ansprüche 11 bis 15 dadurch charakterisiert, dass der pH-Wert der Lösung auf 3,2 bis 4,5 eingestellt ist.

17. Arzneimittelzubereitung nach einem der Ansprüche 11 bis 16 dadurch charakterisiert, dass die aktive Substanz Flunisolid oder Budesonid ist.

18. Arzneimittelzubereitung zur Erzeugung treibgasfreier inhalierbarer Aerosole in Form einer Wirkstofflösung, die als Lösungsmittel 96 Volumenprozent Ethanol und 4 Volumenprozent Wasser und die auf 100ml Lösungsmittel 1,667g Flunisolidhemihydrat und 1mg Dinatrium-EDTA enthält und deren pH-Wert auf 4,0 eingestellt ist.

19. Arzneimittelzubereitung zur Erzeugung treibgasfreier inhalierbarer Aerosole in Form einer Wirkstofflösung, die als Lösungsmittel 90 Volumenprozent Ethanol und 10 Volumenprozent Wasser enthält und die auf 100ml Lösungsmittel 1,667g Flunisolidhemihydrat und 1mg Dinatrium-EDTA enthält und deren pH-Wert auf 4,0 eingestellt ist.

20. Arzneimittelzubereitung zur Erzeugung treibgasfreier inhalierbarer Aerosole in Form einer Wirkstofflösung, deren Lösungsmittel zu 96 Volumenprozent aus Ethanol und zu 4 Volumenprozent aus Wasser besteht und die in 100ml Lösungsmittel 1,333g Budesonid und 1mg Dinatrium-EDTA enthält und deren pH-Wert auf 4,0 eingestellt ist.

21. Arzneimittelzubereitung zur Erzeugung treibgasfreier inhalierbarer Aerosole in Form einer Wirkstofflösung, deren Lösungsmittel zu 90 Volumenprozent aus Ethanol und zu 10 Volumenprozent aus Wasser besteht und die auf 100ml Lösungsmittel 1,333g Budesonid und 1mg Dinatrium-EDTA enthält und deren pH-Wert auf 4,0 eingestellt ist.

22. Verwendung einer Arzneimittelzubereitung in Form einer Lösung, dadurch charakterisiert, dass ein pharmakologisch aktiver Wirkstoff zu 0,001 bis 5,0 Gewichtsprozent gegebenenfalls zusammen mit Geschmackstoffen und/oder pharmakolgisch unbedenklichen Hilfsstoffen in einem treibgasfreien Lösungsmittel gelöst ist, das zu wenigstens 70 Volumenprozent aus Ethanol und ansonsten aus Wasser sowie gegebenenfalls Cosolventien besteht zur unmittelbaren Erzeugung treibgasfreier inhalierbarer, lungengängiger Aerosole.

23. Verwendung nach Anspruch 22, dadurch charakterisiert, daß die Arzneimittelzubereitung eine Arzneimittelzubereitung nach einem der Ansprüche 1 bis 21 ist.

24. Verwendung nach Anspruch 22 oder 23, **dadurch gekennzeichnet, daß** die Arzneimittelzubereitung in einem Inhalator verwendet wird, der die Lösung unter hohem Druck über zwei Strahlen, impaktiert aus zwei kleinen Düsenöffnungen, vernebelt und dadurch ein inhalierbares, treibgasfreies Aerosol mit Partikelgrößen von 1-10 Mikrometer generiert.

25. Aerosol bestehend aus inhalierbaren Partikeln, mit einer Größe von 1-10 Mikrometer, herstellbar durch Vernebelung einer Arzneimittelzubereitung in Form einer Wirkstofflösung nach einem der Ansprüche 1-21 durch einen Inhalator, der die Lösung unter hohem Druck über zwei Strahlen, impaktiert aus zwei kleinen Düsenöffnungen, vernebelt.

## Claims

1. Pharmaceutical preparation for producing propellent gas-free inhalable aerosols for administering into the lungs in the form of a solution of active substance, **characterised in that** an active substance selected from among the betamimetics, anticholinergics, antiallergics, PAF-antagonists, steroids and combinations of said active substances in amounts of 0.001 to 5.0 percent by weight, optionally together with flavourings and/or pharmacologically acceptable excipients, is dissolved in a solvent free from propellent gas which consists of at least 70 percent by volume of ethanol, the remainder being water and optionally cosolvents.

2. Pharmaceutical preparation according to claim 1, **characterised in that** the active substance is selected from among the betamimetics, anticholinergics and/or antiallergics.

3. Pharmaceutical preparation according to claim 1 or 2, **characterised in that** the solvent contains at least 85 percent by volume of ethanol.

4. Pharmaceutical preparation according to one of claims 1, 2 or 3, **characterised in that** the solvent contains at least 95 percent by volume of ethanol.

5. Pharmaceutical preparation according to one of claims 1 to 4, **characterised in that** the solvent contains, in addition to the active substance, one or more pharmacologically acceptable adjuvants or flavourings.

6. Pharmaceutical preparation according to claim 5, **characterised in that** the solvent contains at least 85% by volume of ethanol and the pharmaceutical preparation contains a complexing agent.

7. Pharmaceutical preparation according to claim 6, **characterised in that** the solvent contains at least 96% by volume of ethanol and the complexing agent is EDTA or a salt thereof.

8. Pharmaceutical preparation according to one of claims 1 to 7, **characterised in that** the active substance is tiotropium bromide or an acid addition salt thereof.

9. Pharmaceutical preparation according to one of claims 1 to 7, **characterised in that** the active substance is 3-[(hydroxydi-2-thienylacetyl)oxy]-8,8-dimethyl-8-azoniabicyclo[3,2,1]oct-6-ene or an acid addition salt thereof.

10. Pharmaceutical preparation according to one of claims 1 to 5, **characterised in that** the active substance is ipratropium bromide, oxitropium bromide or trospium chloride.

11. Pharmaceutical preparation according to one of claims 1, 3, 4 or 5, **characterised in that** the active substance is a steroid and the pharmaceutical preparation contains an organic or inorganic acid.

12. Pharmaceutical preparation according to claim 11, **characterised in that** the solvent contains at least 96% by volume of ethanol.

13. Pharmaceutical preparation according to claim 11 or 12, **characterised in that** the pharmaceutical preparation contains a complexing agent.

14. Pharmaceutical preparation according to claim 13, **characterised in that** the complexing agent is EDTA or a salt thereof.

15. Pharmaceutical preparation according to one of claims 11, 12, 13 or 14, **characterised in that** the quantity of complexing agent is between 0.1 and 5 mg/100 ml of solvent.

16. Pharmaceutical preparation according to one of claims 11 to 14, **characterised in that** the pH of the solution is adjusted to between 3.2 and 4.5.

17. Pharmaceutical preparation according to one of claims 11 to 16, **characterised in that** the active substance is flunisolide or budesonide.

18. Pharmaceutical preparation for producing propellent gas-free inhalable aerosols in the form of a solution of active substance which contains as solvent 96 percent by volume of ethanol and 4 percent by volume of water, and which contains, per 100 ml of solvent, 1.667 g of flunisolide-hemihydrate and 1 mg of disodium EDTA and the pH of which is adjusted to 4.0.

19. Pharmaceutical preparation for producing propellent gas-free inhalable aerosols in the form of a solution of active substance which contains as solvent 90 percent by volume of ethanol and 10 percent by volume of water, and which contains, per 100 ml of solvent, 1.667 g of flunisolide-hemihydrate and 1 mg of disodium EDTA and the pH of which is adjusted to 4.0.

20. Pharmaceutical preparation for producing propellent gas-free inhalable aerosols in the form of a solution of active substance, the solvent of which consists of 96 percent by volume of ethanol and 4 percent by volume of water, and which contains, per 100 ml of solvent, 1.333 g of budesonide and 1 mg of disodium EDTA and the pH of which is adjusted to 4.0.

21. Pharmaceutical preparation for producing propellent gas-free inhalable aerosols in the form of a solution of active substance, the solvent of which consists of 90 percent by volume of ethanol and 10 percent by volume of water, and which contains, per 100 ml of solvent, 1.333 g of budesonide and 1 mg of disodium EDTA and the pH of which is adjusted to 4.0.

22. Use of a pharmaceutical preparation in the form of a solution, **characterised in that** a pharmacologically active substance according to claim 1 is dissolved, in an amount of from 0.001 to 5.0 percent by weight, optionally together with flavourings and/or pharmacologically acceptable excipients, in a propellent gas-free solvent which consists of at least 70 percent by volume of ethanol, the remainder being water, optionally together with cosolvents, for directly producing propellent gas-free inhalable aerosols for administering to the lungs.

23. Use according to claim 22, **characterised in that** the pharmaceutical preparation is a pharmaceutical preparation according to one of claims 1 to 21.

24. Use according to claim 22 or 23, **characterised in that** the pharmaceutical preparation is used in an inhaler which nebulises the solution under high pressure in two jets, squirted out through two small nozzle openings, thereby generating an inhalable propellent gas-free aerosol with particle sizes from 1 to 10 microns.

25. Aerosol consisting of inhalable particles, with sizes from 1 to 10 microns, which can be produced by nebulising a pharmaceutical preparation in the form of a solution of active substance according to one of claims 1 to 21 through an inhaler which nebulises the solution under high pressure in two jets squirted out through two small nozzle openings.

## Revendications

1. Préparation médicamenteuse pour la production d'aérosols sans gaz propulseur, inhalables, pouvant parvenir dans les poumons sous forme d'une solution de substance active **caractérisée en ce qu'**une substance active du groupe des bêta-mimétiques, des anticholinergiques, des anti-allergiques, des antagonistes de PAF, des stéroïdes ainsi que leurs combinaisons de substances actives est dissoute à raison de 0,001 à 5,0 % en masse éventuellement avec des agents de sapidité et/ou des adjuvants pharmacologiquement acceptables dans un solvant sans gaz propulseur, qui consiste à raison d'au moins 70 % en volume en éthanol et pour le reste en eau ainsi qu'éventuellement en cosolvants.

2. Préparation médicamenteuse selon la revendication 1 **caractérisée en ce que** la substance active est choisie dans le groupe des bêta-mimétiques, des anticholinergiques et/ou des anti-allergiques.

3. Préparation médicamenteuse selon la revendication 1 ou 2 **caractérisée en ce que** le solvant contient au moins 85 % en volume d'éthanol.

4. Préparation médicamenteuse selon l'une des revendications 1, 2 et 3 **caractérisée en ce que** le solvant contient au moins 95 % en volume d'éthanol.

5. Préparation médicamenteuse selon l'une des revendications 1-4 **caractérisée en ce que** la solution contient, outre la substance active, un ou plusieurs adjuvants ou agents de sapidité pharmacologiquement acceptables.

6. Préparation médicamenteuse selon la revendication 5 **caractérisée en ce que** le solvant contient au moins 85 % en volume d'éthanol et la préparation médicamenteuse contient un complexant.

7. Préparation médicamenteuse selon la revendication 6 **caractérisée en ce que** le solvant contient au moins 96 % en volume d'éthanol et le complexant est l'EDTA ou un sel de celui-ci.

8. Préparation médicamenteuse selon l'une des revendications 1-7 **caractérisée en ce que** la substance active est le bromure de tiotropium ou un sel d'addition d'acide de celui-ci.

9. Préparation médicamenteuse selon l'une des revendications 1-7 **caractérisée en ce que** la substance active est le 3-[hydroxydi-2-thiénylacétyl)oxy]-8,8-diméthyl-8-azoniabicyclo[3,2,1]oct-6-ène ou un sel d'addition d'acide de celui-ci.

10. Préparation médicamenteuse selon l'une des revendications 1-5 **caractérisée en ce que** la substance active est le bromure d'ipratropium, le bromure d'oxitropium ou le chlorure de trospium.

11. Préparation médicamenteuse selon l'une des revendications 1, 3, 4 ou 5 **caractérisée en ce que** la substance active est un stéroïde et la préparation médicamenteuse contient un acide organique ou inorganique.

12. Préparation médicamenteuse selon la revendication 11 **caractérisée en ce que** le solvant contient de l'éthanol à raison d'au moins 96 % en volume.

13. Préparation médicamenteuse selon la revendication 11 ou 12 **caractérisée en ce que** la préparation médicamenteuse contient un complexant.

14. Préparation médicamenteuse selon la revendication 13 **caractérisée en ce que** le complexant est l'EDTA ou un sel de celui-ci.

15. Préparation médicamenteuse selon l'une des revendications 11, 12, 13 ou 14 **caractérisée en ce que** la quantité de complexant est de 0,1 à 5 mg pour 100 ml de solvant.

16. Préparation médicamenteuse selon l'une des revendications 11 à 14 **caractérisée en ce que** le pH de la solution est ajusté à 3,2 à 4,5.

17. Préparation médicamenteuse selon l'une des revendications 11 à 16 **caractérisée en ce que** la substance active est le flunisolide ou le budésonide.

18. Préparation médicamenteuse pour la production d'aérosols inhalables sans gaz propulseur sous forme d'une solution de substance active qui contient comme solvant 96 % en volume d'éthanol et 4 % en volume d'eau et pour 100 ml de solvant 1,667 g de flunisolide hémihydraté et 1 mg de EDTA de disodium et dont le pH est ajusté à 4,0.

19. Préparation médicamenteuse pour la production d'aérosols inhalables sans gaz propulseur sous forme d'une solution de substance active qui contient comme solvant 90 % en volume d'éthanol et 10 % en volume d'eau et qui contient pour 100 ml de solvant 1,667 g de flunisolide hémihydraté et 1 mg d'EDTA de disodium et dont le pH est ajusté à 4,0.

20. Préparation médicamenteuse pour la production d'aérosols inhalables sans gaz propulseur sous forme d'une solution de substance active dont le solvant consiste à raison de 96 % en volume en éthanol et à raison de 4 % en volume en eau et qui contient dans 100 ml de solvant 1,333 g de budésonide et 1 mg d'EDTA de disodium et dont le pH est ajusté à 4,0.

21. Préparation médicamenteuse pour la production d'aérosols inhalables sans gaz propulseur sous forme d'une solution de substance active dont le solvant consiste à raison de 90 % en volume en éthanol et à raison de 10 % en volume en eau et qui contient pour 100 ml de solvant 1,333 g de budésonide et 1 mg d'EDTA de disodium et dont le pH est ajusté à 4,0.

22. Utilisation d'une préparation médicamenteuse sous forme d'une solution **caractérisée en ce qu'**une substance pharmacologiquement active selon la revendication 1 est dissoute à raison de 0,001 à 5,0 % en masse, éventuellement avec des agents de sapidité et/ou des adjuvants pharmacologiquement acceptables, dans un solvant sans gaz propulseur qui consiste à raison d'au moins 70 % en volume en éthanol et sinon en eau ainsi éventuellement qu'en cosolvants pour la production immédiate d'aérosols inhalables sans gaz propulseur pouvant parvenir dans les poumons.

23. Utilisation selon la revendication 22 **caractérisée en ce que** la préparation médicamenteuse est une préparation médicamenteuse selon l'une des revendications 1 à 21.

24. Utilisation selon la revendication 22 ou 23 **caractérisée en ce que** la préparation médicamenteuse est utilisée dans un inhalateur qui nébulise la solution sous haute pression par le biais de deux jets, amenés à se rencontrer à partir de deux petites ouvertures de buse, et génère ainsi un aérosol inhalable dans gaz propulseur ayant des tailles de particules de 1-10 micromètre.

25. Aérosol consistant en particules inhalables d'une taille de 1-10 micromètres, pouvant être produit par nébulisation d'une préparation médicamenteuse sous forme d'une solution de substance active selon l'une des revendications 1-21 au moyen d'un inhalateur qui nébulise la solution sous haute pression par le biais de deux jets amenés à se rencontrer à partir de deux petites ouvertures de buse.
